# EUROPEAN PATENT APPLICATION

(11) **EP 2 098 167 A1**
(43) Date of publication of application: **09.09.2009**
(21) Application number: 07850734.0
(22) Date of filing: 17.12.2007
(51) Int. Cl.: A61B 5/157, A61B 5/151, G01N 27/28, G01N 27/327, G01N 27/416

(54) **BIOSENSOR CARTRIDGE, METHOD OF USING BIOSENSOR CARTRIDGE, BIOSENSOR DEVICE AND NEEDLE-INTEGRATED SENSOR**

(30) Priority: 19.12.2006 JP 2006341792; 16.02.2007 JP 2007035931
(71) Applicant: National Institute of Advanced Industrial Science and Technology, Tokyo 100-8921 (JP); Sumitomo Electric Industries, Ltd., Osaka-shi, Osaka 541-0041 (JP)
(72) Inventor: FUJIMURA, Tsuyoshi, Tsukuba-shi Ibaraki 305-8562 (JP); NAKAMURA, Hideaki, Tsukuba-shi Ibaraki 305-8562 (JP); GOTOH, Masao, Tsukuba-shi Ibaraki 305-8562 (JP); KARUBE, Isao, Tsukuba-shi Ibaraki 305-8562 (JP); ISHIKAWA, Tomoko, Tsukuba-shi Ibaraki 305-8562 (JP); KITAMURA, Takahiko, Osaka 554-0024 (JP); KAIMORI, Shingo, Osaka 554-0024 (JP); NAKAJIMA, Hiroto, Osaka 554-0024 (JP); HAYAMI, Hiroshi, Osaka 554-0024 (JP); HOSOYA, Toshifumi, Osaka 554-0024 (JP)
(74) Representative: Setna, Rohan P.
(86) International application number: PCT/JP2007/074246
(87) International publication number: WO 2008/075651

(57) **Abstract**

A biosensor cartridge capable of making the collected amount of a sample required for measurement into a small quantity to reduce a user's burden, and easily collecting and measuring a sample of a puncture hole without requiring the operation of bringing a sample collection opening close to the puncture hole is provided. When one end portion of biosensor chip 11 is pushed against subject M, elastic body 20 is compressed, and puncturing tool 12 can protrude and perform puncturing. Additionally, if a pushing force is weakened, puncturing tool 12 is extracted from subject M by the restoring force of elastic body 20, and blood (sample) D flows out of the puncture hole. In this case, since the puncture hole, and sample collection opening 13 provided at biosensor chip 11 are contained in enclosed half-open space 23 formed by elastic body 20, even a small amount of blood (sample) D can also be easily collected. Additionally, elastic body 20 is formed with a groove 28 which forms a ventilation passage, so that enclosed half-open space 23 can be opened to the atmosphere.

## Description

### Technical Field

The present invention relates to a biosensor cartridge, for example, a biosensor cartridge which conducts measurement and analysis of a chemical substance using a reagent contained in a hollow reaction portion of a chip. Additionally, the present invention relates to a biosensor used for a simple body fluid measuring device which simply analyzes characteristics of the substance, and specifically, to a needle integral sensor which has a puncturing needle for sample collection and a sensor which are made integral and which is adapted such that a subject can perform measurement by himself.

### Related Art

In related art, for example, a biosensor chip which detects the concentration of glucose in blood is known (for example, refer to Patent Document 1).
Fig. 18 is an exploded perspective view showing a glucose sensor described in Patent Document 1. As shown in Fig. 18, glucose sensor 100 that is a biosensor has counter electrode 101 and working electrode 102. Counter electrode 101 assumes a hollow needle shape which is half-cut in its longitudinal direction, and tip portion 103 thereof is obliquely cut in the shape of an injection needle for easy puncturing. Insulating layers 104 and 104' which generally serve also as an adhesive layer, for example, epoxy resin adhesive, silicone-based adhesive, or glass is applied to the half-cut face, and working electrode 102 is attached to the half-cut face via insulating layers 104 and 104'. Working electrode 102 is a flat-plate-shaped member where glucose oxidase (GOD) is immobilized, and is bonded to counter electrode 101 with the face of a so-called immobilized GOD 105 where GOD is immobilized being directed inward.
Accordingly, a subject is punctured by tip section 103 of needlelike counter electrode 101 to collect blood; and the reaction between the collected blood and immobilized GOD 105 is detected by working electrode 102, thereby performing the quantitative determination of glucose.

Additionally, a biosensor in which a biosensor chip and a lancet are integrated is disclosed (for example, refer to Patent Document 2).
Fig. 19A is a perspective view showing a sensor described in Patent Document 2, and Fig. 19B is an exploded perspective view of the sensor. As shown in Fig. 19, lancet integral sensor 110 has chip body 111, lancet 113, and protective cover 115. Chip body 111 has cover 111a and substrate 111b openably and closably, and an inner surface of cover 111a is formed with internal space 112. Internal space 112 assumes a shape which can house lancet 113 movably.

A needle 114 provided at the tip of the lancet 113 is able to protrude and retract from an opening 112a formed at a front end of the internal space 112 of the chip body 111 with movement of the lancet 113. The shape of the internal space 111a is bent so as to become slightly narrower than lancet 113 at the end in which projection 113a is located, and lancet 113 is locked to chip body 111 by a mutual pressing force or frictional force. Protective cover 115 has tube portion 115a into which needle 114 is inserted and fitted, and tube portion 115a can also be housed inside chip body 111 with movement of needle 114. Accordingly, in the state before use, protective cover 115 is put on needle 114 to protect needle 114 and prevent a user from being injured accidentally. In addition, substrate 111b is provided with a pair of electrode terminals 116, and these terminals can be electrically connected to a biosensor device (not shown).

When being used, protective cover 115 is removed, and lancet 113 is pushed to make needle 114 protrude from chip body 111. After a subject is punctured in this state, the needle 114 is housed in the chip body 111, and the opening 112a provided at the front end of the chip body 111 is brought close to a puncture hole, whereby blood which has flowed out is collected.

Additionally, illustration of a biosensor disclosed in Document 3 is omitted in the drawing. However, the biosensor has a needle integral structure including a detecting portion in which a detecting chip is composed of a test paper, and has the structure of using a decompression pump in order to collect blood to the detecting portion after a subject is punctured.
Patent Document 1: Unexamined Japanese Patent Application Publication No. Hei2-120655
Patent Document 2: Pamphlet of International Publication WO 02/056769
Patent Document 3: Unexamined Japanese Patent Application Publication No. 2002-085384

### Disclosure of the Invention

### Problems to be solved by the Invention

However, since needlelike counter electrode 101 and working electrode 102 are formed so as to be stuck together in glucose sensor 100 described in Patent Document 1, the diameter of a puncture needle becomes nearly equal to the width of glucose sensor 100, and becomes large. For this reason, there is a problem that the amount of blood collected increases, and pain during puncturing becomes large, and consequently, user's burden becomes acute.
Additionally, lancet integral sensor 110 described in Patent Document 2 has a structure which absorbs the blood which has flowed out of a puncture hole from opening 112a. However, the structure is complicated.
Furthermore, even the biosensor described in Patent Document 3 faces the problem that the structure as a biosensor device is complicated, such as using a decompression pump.

Additionally, the lancet integral sensor drives needle 114 within internal space 112 which houses a sample. Therefore, the size of internal space 112 becomes a size required for driving of needle 114, and the amount of blood required for measurement increases more than that of a measurement kit in which a sensor and a puncturing needle are separate. This makes a needle thick or makes a needle pierce a subject deeply, thereby increasing the pain at the time of sampling of the subject. Additionally, it is difficult that a lancet integral type sensor which integrally has the sensor body and the drive mechanism of a needle is miniaturized.

Meanwhile, a sensor in which a cavity containing blood is provided separately from a lancet storage space within a sensor body is also suggested in Patent Document 2. In this case, since the size of the cavity can be set independently of the lancet, it can be considered that it becomes possible to reduce the amount of blood collected. However, even though the piercing position by the lancet and the inlet of the cavity are disposed in different positions, a mechanism for effectively containing blood discharged to the surface of skin into the cavity, and blood adhered to the needle is not disclosed at all.

The invention is made in view of the aforementioned problems, and the object thereof is to provide a biosensor cartridge capable of making the collected amount of a sample required for measurement into a small quantity to reduce a user's burden, and easily collecting and measuring a sample of a puncture hole without requiring the operation of bringing a sample collection opening close to the puncture hole, and performing exact measuring.
Additionally, the object of the invention is to provide a needle integral sensor which is formed as a needle integral sensor in which a puncturing needle is fixed to a sensor and which can reduce the size of a reaction space (cavity) of a sample and the amount of collected blood required for measurement, and can effectively introduce blood discharged to a skin surface into a cavity even in a position where the puncturing position of the needle, and an inlet of the cavity are separated from each other.

### Means for solving the Problems

In order to achieve the aforementioned object, according to a first aspect of the invention, there is provided a biosensor cartridge including:
a biosensor chip, and
a puncturing tool fixed to a portion of the biosensor chip and having a tip protruding therefrom, wherein
an elastic body having a through hole which contains a sample collection opening provided at a tip of the biosensor chip and a puncture hole formed in a subject by the puncturing tool is provided at a tip of the biosensor chip, and
the elastic body includes a ventilation passage which passes through between an internal wall surface which forms the through hole and an external wall surface of the elastic body.

In the biosensor cartridge configured in this way, when the one end portion of the biosensor chip is pushed against a subject, the elastic body provided at a portion of the biosensor chip is compressed, and the puncturing tool protrudes. Thus, the subject can be punctured. Additionally, if a pushing force is weakened, the puncturing tool is extracted from the subject by the restoring force of the elastic body, and the sample flows out of the puncture hole, and the sample can be collected.
Additionally, according to this biosensor cartridge, the puncturing tool is prevented from protruding from the tip face of the elastic body before use, and thereby protection of the puncturing tool and protection of a user can be achieved. Moreover, even when being discarded after use, the puncturing tool is prevented from protruding from the tip face of the elastic body, and thereby, the tool can be safely and properly disposed.

Further, according to a second aspect of the invention, there is provided the biosensor cartridge of the first aspect, wherein
the sample collection opening provided at the tip of the biosensor chip and the puncture hole formed in the subject by the puncturing tool are connected together by the through hole.
In the biosensor cartridge configured in this way, since the puncture hole, and the sample collection opening provided at the tip of the biosensor chip are connected in the space formed by the elastic body at the time of puncturing, even a small amount of sample can be collected easily.

Further, according to a third aspect of the invention, there is provided the biosensor cartridge of the first or second aspect, wherein
the elastic body is configured such that the internal diameter of the hole on the side which touches the subject is made larger than the internal diameter of the hole on the side which touches the biosensor chip.

In the biosensor cartridge configured in this way, the size of the through hole for sample collection is made large. Thus, at the time of puncturing, the puncture hole and the space of the elastic body can reliably maintain the positional relationship required for sample collection. Additionally, unexpected contact between a sample and the elastic body is avoided, and the sample collected is guided to the biosensor chip without being oozed into a contact portion between a subject and the elastic body.

Further, according to a fourth aspect of the invention, there is provided the biosensor cartridge of the third aspect, wherein
the elastic body is configured such that the internal wall surface which forms the through hole is raised toward the tip of the elastic body on the side which touches the subject. Thereby, a sample in the through hole first contacts the surface whose internal wall surface is raised, and is guided to above the through hole.
Further, according to a fifth aspect of the invention, there is provided the biosensor cartridge of any one of the first to fourth aspects, wherein
the ventilation passage is a groove provided at the end face of the elastic body on the side of the subject.

In the biosensor cartridge configured in this way, the ventilation passage can be formed in a contact portion between a subject and the elastic body by the groove. Additionally, this groove is very easily formed, and does not complicate manufacture of the elastic body.

Further, according to a sixth aspect of the invention, there is provided the biosensor cartridge of any one of the first to fourth aspects, wherein
the ventilation passage is an open through hole formed substantially along a radial direction of the elastic body.

In the biosensor cartridge configured in this way, this open through hole does not use a subject as its component but is composed of only the elastic body. Thus, setting of a cross-sectional shape or the like is performed easily, and is not influenced by the state of the subject.

Further, according to a seventh aspect of the invention, there is provided the biosensor cartridge of any one of the first to sixth aspects, wherein
a plurality of the ventilation passages are formed in the elastic body, and are arranged so as to be symmetrical in the radial direction of the elastic body with the axial center of the elastic body as a center.

In the biosensor cartridge configured in this way, the ventilation passages are arranged so as to be symmetrical in the radial direction of the elastic body with the axial center of the elastic body as a center. Thereby, the elastic force of the elastic body can be made symmetrical in the radial direction of the elastic body with the axial center of the elastic body as a center, and for example, when the elastic body is compressed, the balance of elasticity can be maintained without deviation.

Further, according to an eighth aspect of the invention, there is provided the biosensor cartridge of any one of the first to seventh aspects, further including:
a drive mechanism which punctures a subject with the puncturing tool.

In the biosensor cartridge configured in this way, the puncturing tool punctures a subject by a drive mechanism. Thereby, it is possible to shorten puncturing time and alleviate a pain when a sample is collected.

Further, according to a ninth aspect of the invention, there is provided the biosensor cartridge of any one of the first to eighth aspects, wherein
the elastic body has stickiness.
Particularly, it is preferable that the surface which touches a subject has stickiness. A case where the raw material of the elastic body itself has stickiness, a case where a gluing agent is kneaded into the elastic body, a method of performing coating with a gluing agent, and the like can be exemplified.

In the biosensor cartridge configured in this way, since the elastic body has stickiness, the elastic body can be brought into close contact with a subject, and deviation of a puncturing position can be prevented, and collection of a sample can be ensured.

Further, according to a tenth aspect of the invention, there is provided the biosensor cartridge of any one of the first to ninth aspects, wherein
the elastic body is fixed to the tip of the biosensor chip with its own stickiness, fixed to the tip of the biosensor chip with an adhesive, or fixed to the tip of the biosensor chip with a double-sided tape.

In the biosensor cartridge configured in this way, the elastic body can be reliably attached to the tip of the biosensor chip. At this time, when a gluing agent is used and when a gap is formed between the elastic body and the biosensor chip, adhesion can be ensured by applying the adhesive so that this gap is filled, and leakage of a collected sample can be prevented.

Further, there is provided a method of using a biosensor cartridge of any one of the first to tenth aspects, the method including the steps of:
pushing the elastic body against the subject to compress the elastic body to puncture the subject,
pulling out a puncturing tool from the subject by a restoring force of the elastic body, and
performing sample collection while the through hole is opened to the atmosphere.

In the method of using such a biosensor cartridge, when the one end portion of the biosensor chip is pushed against a subject, the elastic body provided at the one end portion of the biosensor chip is compressed, and the puncturing tool protrudes. Thus, the subject can be punctured. Thereafter, the puncturing tool is pulled out by the restoring force of the elastic body, and a sample which has flowed out of a puncture hole within a space formed by the elastic body is collected from the sample collection opening. Thus, the sample can be reliably collected after puncturing without positioning the sample collection opening in the puncture hole.

Further, there is provided a biosensor device including:
the biosensor cartridge of any one of the first to tenth aspects, and
a measuring instrument which is connected to detecting electrodes of the biosensor cartridge and obtains information on a sample which has been collected.

In the biosensor device configured in this way, a sample is collected by the aforementioned biosensor cartridge. Thus, puncturing and sample collecting can be performed by a series of operation, collecting of a sample can be easily and reliably ensured without being required to align the sample collection opening of the biosensor chip with the puncture hole unlike a related art. Additionally, information on a sample is transmitted to the measuring instrument via the detecting electrodes, so that measurement can be easily performed in a short time, and a burden on a subject can be reduced.

Additionally, in the needle integral sensor in which the puncturing needle is fixed to the sensor, in order to perform piercing required to discharge a liquid sample such as blood from a subject to a skin surface, the puncturing needle is fixed in a protruding state. For this reason, although the positional relationship that the tip of puncturing needle and the inlet of a sample reaction space are separated from each other is inevitably obtained, this can be solved by providing a flow passage through which a liquid sample can flow from the tip of the puncturing needle to the inlet of a sample reaction space. Thus, the present inventors have variously examined a configuration in which a flow passage forming body which forms a flow passage through which a liquid sample can flow from the tip of the puncturing needle to the inlet of a sample reaction space is attached, and a liquid sample is introduced into the sample reaction space through the flow passage, and has completed the invention.

That is, there is provided a needle integral sensor including:
a needle integral sensor body integrally having a puncturing needle which discharges a liquid sample from a subject, a reaction portion which contains the liquid sample, and a detecting portion which detects the result of the reaction portion; and
a flow passage forming body in which a through hole serving as a flow passage from an abutting portion of the subject to the reaction portion is provided, and in which the puncturing needle is inserted into the through hole, wherein
the flow passage forming body is formed with a ventilation passage which communicates the through hole and the outside of the flow passage forming body with each other.

The needle integral sensor of the invention is used so as to drive not the puncturing needle independently, but the needle integral sensor body, puncture the skin of a subject, and discharge a liquid sample. Although the material of the flow passage forming body is not particularly limited, the material may be a rigid body, may be an elastic body, and may be a viscoelastic body. However, preferably, the flow passage forming body is an elastic body or viscoelastic body which enables the puncturing needle to project and retract from the through hole by deformation. This is because the puncturing needle which has punctured a skin is pulled out by the elastic restoring force of the flow passage forming body itself, and thereby, the measuring instrument has only to be provided with a drive mechanism for performing puncturing with a puncturing needle.

The ventilation passage may be formed in a certain position of the flow passage forming body as long as it is provided so as to allow the through hole and the outside of the flow passage forming body to communicate with each other. Accordingly, the ventilation passage may be a communication hole provided so as to pass through the peripheral wall surface of the through hole which constitutes the through hole in a proper position of the peripheral wall surface, may be a recess recessed in the attachment portion of the sensor body, may be a gap formed in the attachment portion between the sensor body and the flow passage forming body in a state where the needle integral sensor body is attached, may be a cutout provided in the subject abutting surface of the flow passage forming body so that the through hole and the outside of the flow passage forming body communicates with each other in a state of abutting on subject, or may be combinations of them.

Preferably, the ventilation passage is provided in an attachment portion of the sensor body in the flow passage forming body. Here, the attachment portion of the sensor body means a portion that the tip portion of the sensor body and the flow passage forming body probably touch if any ventilation passage is not formed when the puncturing needle is inserted into the through hole, means a joined portion, for example, when the flow passage forming body is joined to the sensor body by bonding, welding, or the like, and means a fitting portion when being attached by fitting. Accordingly, the case where the attachment portion is provided with the ventilation passage includes, for example, a case where a portion of the surface of the flow passage forming body to which the needle integral sensor body is attached (hereinafter referred to as "sensor body attachment surface") is cut out so as to communicate with the outside of the flow passage forming body, a case where the sensor body attachment surface is formed with a groove extending to the outer peripheral surface of the flow passage forming body, and a case where the sensor body attachment surface itself is formed as a rough surface which allows inflow and outflow of air. Additionally, in a case where the flow passage forming body and the sensor body are attached by fitting, the ventilation passage may be formed by a communication hole which passes through the wall surface of the flow passage forming body which becomes an external fitting portion of the tip of the sensor body or by the clearance between the flow passage forming body and the sensor body in the case of fitting.

In a case where the ventilation passage is provided in the attachment portion of the sensor body, preferably, the ventilation passage is provided so that the reaction portion inlet is located midway of the ventilation passage or in the vicinity of the ventilation passage inlet. Specifically, in a case where the reaction portion inlet is arranged within the through hole formed in the flow passage forming body, it is preferable that the inlet of the ventilation passage communicating with the through hole is arranged in a position closer to the reaction portion inlet than the puncturing needle. Additionally, in a case where the reaction portion inlet is not arranged within the through hole, it is preferable to provide the ventilation passage so that the reaction portion inlet is arranged midway of the ventilation passage which leads to the flow passage forming body from the through hole.

It is preferable that the ventilation passage is provided with a liquid outflow preventing mechanism. Since the through hole serving as a flow passage for a liquid sample and the ventilation passage communicate with each other, the liquid sample is prevented from leaking out to the outside of the flow passage forming body through the ventilation passage. As the liquid outflow preventing mechanism provided in the ventilation passage, for example, a curved portion may be provided at a midway of the ventilation passage, and a protective wall for prevention of liquid outflow may be provided in the flow passage forming body.

Additionally, it is more preferable that a liquid sample passing through the through hole be made to flow into the reaction portion more preferentially than flowing through the ventilation passage. This is required particularly when the through hole is provided near the reaction portion inlet and the sensor body attachment portion. Making a liquid sample flow into the reaction portion more preferentially than the ventilation passage can be achieved by suitably setting the size of the ventilation passage inlet and the reaction portion inlet, the positional relationship between the ventilation passage and the inlet of the reaction portion, and the like. However, preferably, a surfactant is applied in the vicinity of an inlet of the reaction portion. The surfactant may be applied not only to the reaction portion inlet but to a portion into which a liquid sample flow preferentially.

### Advantage of the Invention

According to the invention, one end of the biosensor chip is provided with the elastic body. Thus, the puncturing tool is pulled out by the restoring force of the elastic body after puncturing, and a sample can be suitably collected from a space formed by the elastic body. Thus, even a small amount of sample can be easily collected by the sample collection opening. Additionally, according to the invention, a space where a sample is collected has a ventilation passage which can be opened to the atmosphere. Thus, the sample can be stably introduced into the sample collection opening, and a method of using a biosensor cartridge and a biosensor device capable of carrying out a reliable test can be provided.

Additionally, the needle integral sensor of the invention includes a flow passage forming body which can guide a liquid sample discharged by puncturing to the inlet of the reaction portion disposed so as to be separated from the tip of the puncturing needle, and the flow passage forming body is formed with a ventilation passage which allows a through hole serving as a flow passage and the outside of the flow passage forming body to communicate with each other. Thereby, the liquid sample discharged by puncturing can be effectively introduced into the reaction portion. Thus, the amount of collection of the liquid sample required for measurement can be reduced.

### Brief Description of the Drawings

Fig. 1A is an explanatory view of a schematic longitudinal section in an embodiment of a biosensor cartridge according to the invention, and Fig. 1B is an explanatory view of a schematic horizontal section in the embodiment of the biosensor cartridge according to the invention.
Fig. 2 is an exploded perspective view showing the junction between an elastic body and a biosensor chip in the embodiment of the biosensor cartridge according to the invention.
Fig. 3 is a cross-sectional view showing the junction between the elastic body and the biosensor chip in the embodiment of the biosensor cartridge according to the invention.
Fig. 4 is a schematic plan view showing the embodiment of the biosensor device according to the invention.
Figs. 5A to 5C are explanatory views showing the operation of measuring blood sugar level using the biosensor device according to the invention.
Fig. 6A is an explanatory view showing another embodiment of the biosensor cartridge according to the invention, and Fig. 6B is an explanatory view showing another embodiment of the biosensor cartridge according to the invention.
Fig. 7 is a cross-sectional view showing the junction between the elastic body and the biosensor chip in another embodiment of the biosensor cartridge according to the invention.
Fig. 8 is a cross-sectional view showing the configuration of a needle integral sensor of one embodiment of the invention.
Figs. 9A and 9B are a plan view (Fig. 9A) and a cross-sectional view (Fig. 9B) showing an example of a detecting portion used for the needle integral sensor shown in Fig. 8.
Fig. 10 is a perspective view showing the configuration of a flow passage forming body used for the needle integral sensor of one embodiment of the invention.
Fig. 11 is a schematic view showing the configuration of an example of a measuring device mounted with the needle integral sensor of this embodiment.
Fig. 12 is a view for explaining the method of using the needle integral sensor of this embodiment.
Fig. 13 is a cross-sectional view showing another embodiment of the needle integral sensor of the invention.
Fig. 14 is a cross-sectional view showing another embodiment of the needle integral sensor of the invention.
Fig. 15 is a view showing a still another embodiment of the flow passage forming body used for the needle integral sensor of the invention.
Fig. 16 is a cross-sectional view showing the configuration of the needle integral sensor to which the flow passage forming body shown in Fig. 15 is attached.
Fig. 17 is a cross-sectional view showing a still further embodiment of the needle integral sensor of the invention.
Fig. 18 is an exploded perspective view showing a biosensor chip of the related art.

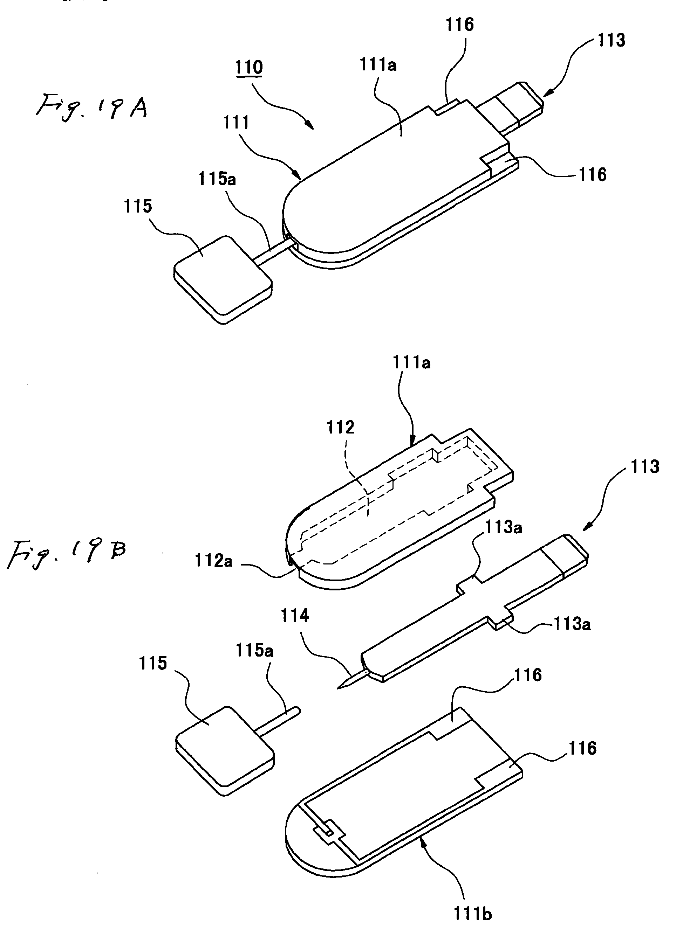

Fig. 19A is a perspective view showing the biosensor chip of the related art, and Fig. 19B is an exploded perspective view showing the biosensor chip of the related art.

### Description of Reference Numerals and Signs

- 10:: BIOSENSOR CARTRIDGE
- 11:: BIOSENSOR CHIP
- 11a:: ONE END PORTION
- 12:: PUNCTURING TOOL
- 12a:: TIP
- 13:: SAMPLE COLLECTION OPENING
- 18a, 18b:: DETECTING ELECTRODE
- 20:: ELASTIC BODY
- 22:: THROUGH HOLE
- 21:: TIP FACE (SURFACE TOUCHING SUBJECT)
- 23:: ENCLOSED HALF-OPEN SPACE (SPACE)
- 24:: GLUING AGENT
- 25:: SURFACE TOUCHING BIOSENSOR CHIP
- 26:: RAISED PORITON
- 28:: GROOVE (VENTILATION PASSAGE)
- 29:: OPEN THROUGH HOLE (VENTILATION PASSAGE)
- 30:: BIOSENSOR DEVICE
- 31:: MEASURING INSTRUMENT
- D:: BLOOD (SAMPLE)
- M:: SUBJECT
- 201:: DETECTING PORTION
- 202:: PUNCTURING NEEDLE
- 203, 203':: REACTION PORTION
- 203a:: REACTION PORTION INLET
- 210:: SENSOR BODY
- 220, 220', 220", 225, 230:: FLOW PASSAGE FORMING BODY
- 221, 221':: INSERTION PORTION
- 222:: THROUGH HOLE
- 223, 223', 226:: VENTILATION PASSAGE

### Best Mode for Carrying Out the Invention

Hereinafter, an embodiment according to the invention will be described in detail with reference to the drawings.

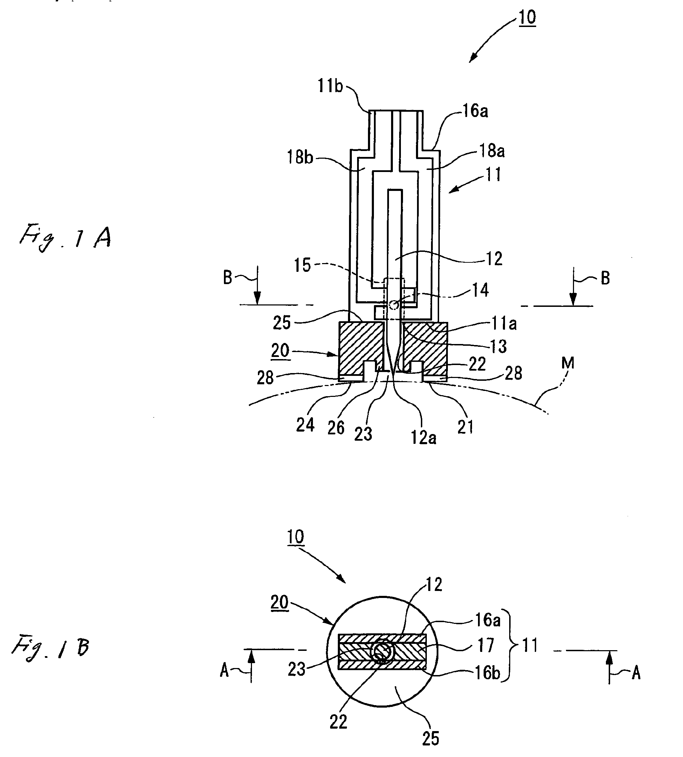

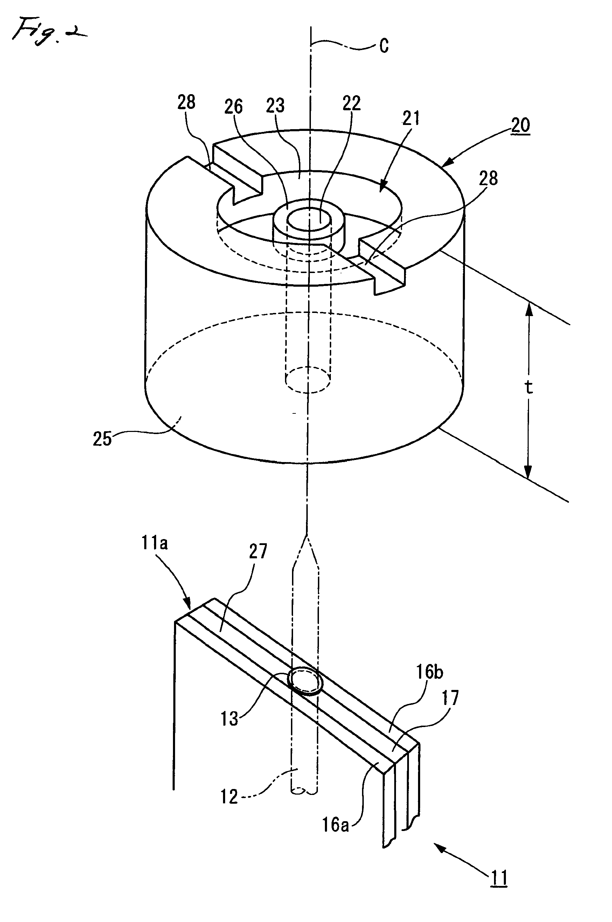

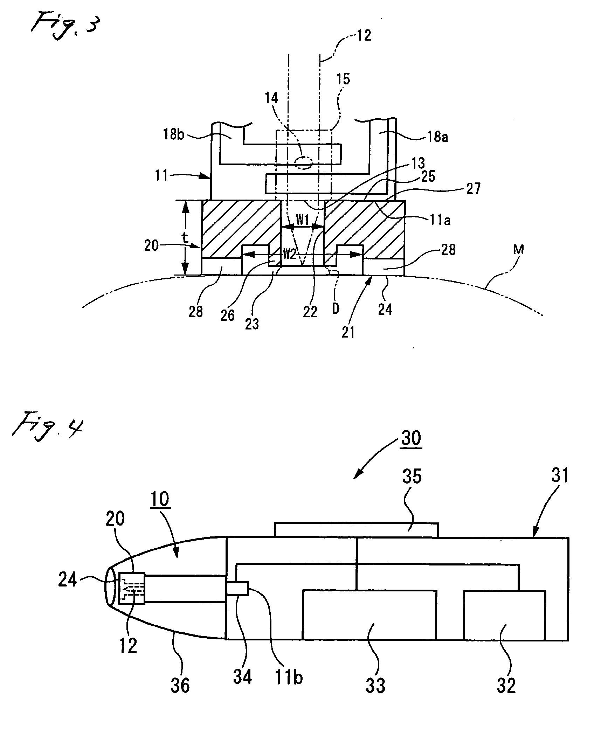

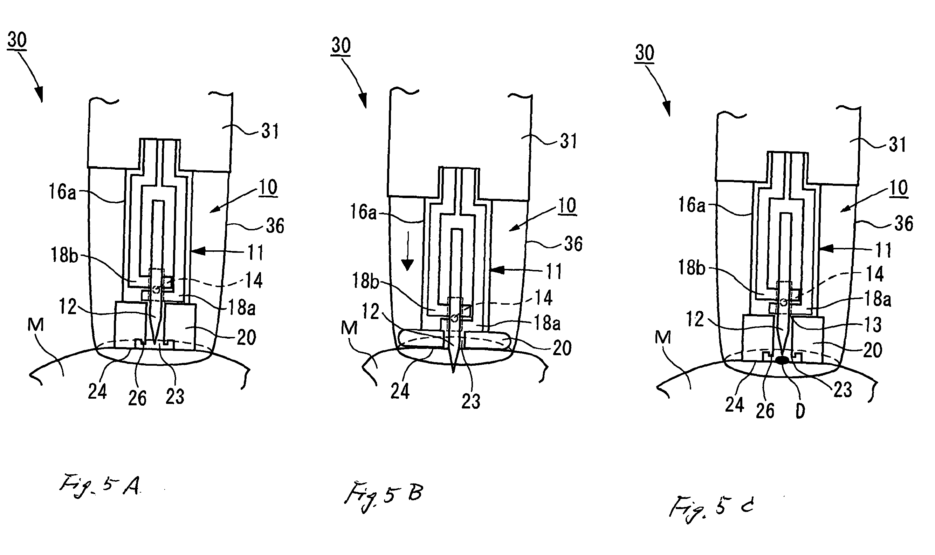

Fig. 1A is a longitudinal sectional view (sectional view of an A-A position in Fig. 1B) when essential portions of an embodiment according to a biosensor cartridge of the invention are seen from the side, and Fig. 1B is a horizontal sectional view (sectional view of a B-B position in Fig. 1A) showing essential portions of the embodiment according to the biosensor cartridge of the invention. Fig. 2 is an exploded perspective view showing an example of the junction between a biosensor chip and an elastic body. Fig. 3 is a cross-sectional view of the junction between the elastic body and the biosensor chip. Fig. 4 is a configuration view showing an embodiment according to a biosensor device of the invention. Figs. 5A to 5C are a series of explanatory views showing collecting operation of a sample using the biosensor device according to the invention.

As shown in Figs. 1A and 1B, and Fig. 2, biosensor cartridge 10 that is an embodiment of the invention has biosensor chip 12, puncturing tool 12 which is fixed to one end portion 11a of biosensor chip 11, and from which a tip 12a protrudes, and electric body 20. That is, by pushing the one end portion against subject M, elastic body 20 which forms enclosed half-open space 23 which contain sample collection opening 13 provided at tip 11a of biosensor chip 11, and a puncture hole formed in subject M by puncturing tool 12 is provided at tip 11a of biosensor chip 11.

In this biosensor cartridge 10, as shown in Figs. 2 and 3, surface 25 which touches biosensor chip 11 of elastic body 20, and surface 11a which touches elastic body 20 of biosensor chip 11 are suitably joined together.

Elastic body 20 includes grooves 28 which forms a ventilation passage which passes through a region between an internal wall surface which defines enclosed half-open space 23 composed of through hole 22 through which puncturing tool 12 passes, and an external wall surface of elastic body 20.

In addition, in the invention, it is desirable that puncturing tool 12 names a needle, a lancet needle, a cannula, etc. generically and is made of a biodegradable material.

Biosensor chip 11 has two substrates 16a and 16b which face each other, and spacer layer 17 which is sandwiched between two substrates 16a and 16b. The surface of at least one substrate 16a of two substrates 16a and 16b on the side of spacer layer 17 is provided with detecting electrodes 18a and 18b, and one end portions (lower ends in Fig. 1A) of the detecting electrodes are bent in an L shape in directions in which they face each other, and holds a predetermined interval therebetween.

As shown in Fig. 3, hollow reaction portion 15 is formed by two substrates 16a and 16b and spacer layer 17 from tip 11a of biosensor chip 11 to a portion where two detecting electrodes 18a and 18b face each other. The tip (lower end in Fig. 3) of hollow reaction portion 15 is provided with sample collection opening 13 through which blood D (refer to Fig. 5C) as a sample collected by puncturing the subject M (refer to Fig. 5) with puncturing tool 12 is introduced into hollow reaction portion 15.

That is, in hollow reaction portion 15, both upper and lower surfaces are formed by substrates 16a and 16b and detecting electrodes 18a and 18b, and a rectangular shape is formed with spacer layer 17 cut away in a predetermined shape as a side wall. For this reason, in hollow reaction portion 15, detecting electrodes 18a and 18b are exposed, and reagent 14 which immobilizes, for example, an enzyme and a mediator, and reacts with glucose in blood D to generate an electric current is provided immediately above or in the vicinity of detecting electrodes 18a and 18b in hollow reaction portion 15. Accordingly, hollow reaction portion 15 becomes a portion where blood D such as blood collected from sample collection opening 13 biochemically reacts with reagent 14.

As the material for substrates 16a and 16b and spacer layer 17, a film that is an insulating material is selected. As the insulating material, ceramics, glass, paper, a biodegradable material (for example, polylactate microorganism production polyester or the like), thermoplastic resins such as a polyvinyl chloride, polypropylene, polystyrene, polycarbonate, acrylic resin, polypbutylene terephthalate, and polyethylene terephthalate (PET), thermoset resin such as epoxy resin, and plastic materials such as UV curable resin can be exemplified. Plastic materials such as polyethylene terephthalate are preferable from the viewpoint of mechanical strength, pliability, easiness of machining and production of chips, and the like. The typical PET resin includes Melinex and Tetoron (these are trade names and made by Teijin DuPont Films Japan Limited), Rumirer (trade name and made by Toray Industries, Inc.), etc.

As reagent 14, enzymes and electron acceptors such as glucose oxidase (GOD), glucose dehydrogenase (GDH), cholesterol oxidase, and uricase are exemplified.
For example, in the case of a glucose biosensor chip which measures the amount of glucose in blood, this portion is formed with a glucose oxidase layer, a glucose oxidase electron acceptor (mediator) mixture layer, a glucose oxidase albumin mixture layer, or a glucose oxidase electron acceptor-albumin mixture layer. These layers may be formed using enzymes other than a glucose oxidase, for example, glucose dehydrogenase or the like. Additionally, a buffering agent, a hydrophilic polymer, etc. as additive agents may be included into the reagent.

As shown in Figs. 2 and 3, as elastic body 20 attached to tip 11a of biosensor chip 11, for example, a cylindrical elastic body which has through hole 22 for forming enclosed half-open space 23 in a central portion can be exemplified. Through hole 22 is larger than the external diameter of puncturing tool 12 so that puncturing tool 12 is inserted therethrough.
Elastic body 20 is configured such that internal diameter W2 of through hole 22 that is enclosed half-open space 23 on the side which touches subject M is larger than internal diameter W1 of a hole on the side which touches biosensor chip 11.

By making the size of through hole 22 large (to internal diameter W2) and by configuring so-called through hole 22 like a reception opening having raised portion 26 which is formed by raising a wall surface around through hole 22, blood D which has flowed into enclosed half-open space 23 can be first brought into positive contact with raised portion 26 around through hole 22, and can be guided to through hole 22. As such, since the portion of blood D which touches the internal wall surface of enclosed half-open space 23 is configured so as to become a region near through hole 22, blood D can be prevented from being oozed out to a contact portion between tip face 21 of elastic body 20, and subject M.

Additionally, the thickness t of elastic body 20 is set to a thickness such that even the tip of puncturing tool 12 can be surely covered.

In addition, the material for elastic body 20 is not particularly limited so long as it has elasticity. However, rubber such as silicone, urethane, and acrylic rubber, rubber or sponge including a polymer simple substance or a copolymerized polymer such as ethylene, and styrene, polyolefine such as polyethylene and polypropylene, polyester such as polyethylene terephthalate and polybutylene terephthalate, and fluororesin such as PFA that is a copolymer of polytetrafluoroethylene and perfluoro alkoxy ethylene, and polyfluoroethylene, etc. can be utilized.
Elastic body 20 may be solid or hollow as long as it has a configuration in which a ventilation passage is formed.

It is desirable that tip face 21 that is the face of elastic body 20 which touches subject M is made of a material having stickiness such as silicone rubber and acrylic rubber, or the elastic body has gluing agent 24, or is coated with gluing agent 24. The gluing agent 24 is not limited unless elasticity is impaired. Thereby, the adhesion between elastic body 20 and subject M can be improved, deviation from a puncturing position can be prevented, and enclosed half-open space 23 can be surely formed. Additionally, it is desirable that the inner peripheral surface of the through hole 22 is formed using a hydrophilic material, or at least the inner peripheral surface is subjected to hydrophilic treatment. Thereby, blood D to be collected can be easily passed through the through hole, and even a small amount of blood D can be surely collected.

As shown in Fig. 2, tip 11a of biosensor chip 11 and upper face 25 (bottom in Fig. 2) of elastic body 20 are suitably joined together, and two grooves 28 are formed at tip face 21 (top in Fig. 2) 13 of elastic body 20 in directions in which they become symmetrical to each other with axial center C of elastic body 20 therebetween. Even when the tip face 21 is closed by subject M, grooves 28 can form ventilation passages to open enclosed half-open space 23 to the atmosphere.
In addition, as for elastic body 20, an internal wall surface which forms a through hole and an external wall surface which touches a subject may not be made of the same material, but it is preferable from the viewpoint of surely collecting blood that the external and internal wall surfaces be formed integrally.

As shown in Fig. 3, it is desirable that biosensor chip 11 and elastic body 20 are surely fixed together with adhesive 27. At this time, when a gap is formed between elastic body 20 and biosensor chip 11, adhesion can be ensured by applying the adhesive so that this gap is filled. Additionally, blood D collected from a junction can be prevented from leaking.
In addition, when the adhesive is applied to biosensor chip 11 and the contact surface of elastic body 20, it is necessary to be careful so that the adhesive does not protrude into a portion which becomes a flow passage for blood D, and the inside of biosensor chip 11.

Next, the biosensor device according to the invention will be described.
The configuration of biosensor device 30 using the above-described biosensor cartridge 10 is shown in Fig. 4.
As shown in Fig. 4, biosensor device 30 has aforementioned biosensor cartridge 10, measuring instrument 31 which is connected to detecting electrodes 18a and 18b of biosensor cartridge 10, and obtains information on the collected blood D, and protective cap 36 of the biosensor cartridge. In addition, the configuration of biosensor cartridge 10 is as described above. Thus, the same parts common to those of the aforementioned biosensor cartridge 10 are denoted by the same reference numerals, and the description thereof is omitted.

Measuring instrument 31 includes power source 32, control device 33, terminal insertion portion 34, and display unit 35, and these are connected to each other. Rear end 11b of biosensor chip 11 in biosensor cartridge 10 is inserted into and fixed to terminal insertion portion 34, and detecting electrodes 18a and 18b exposed to rear end 11c of biosensor chip 11 are electrically connected to each other. Biosensor system 30 is small-sized, and is, for example, a handy type which enables a subject to grip with a single hand.

Next, a method of using the biosensor device will be described with reference to Figs. 5A to 5C, taking a case where the blood sugar level is measured using biosensor device 30 as an example.
First, as shown in Fig. 4, rear end 11b of biosensor chip 11 of biosensor cartridge 10 is inserted into, fixed to, and electrically connected to terminal insertion portion 34 of measuring instrument 31. Power source 32 of biosensor system 30 is switched on, and whether or not the power source is normally started is confirmed.

As shown in Fig. 5A, biosensor device 30 is held, protective cap 36 is pushed against a subject to congest a puncturing place, and elastic body 20 attached to tip 11a of biosensor cartridge 10 is brought into contact with a blood collecting place of subject M. Since the tip face of elastic body 20 is coated with gluing agent 24, positional deviation can be prevented in the subsequent operation.

Next, as shown in Fig. 5B, biosensor cartridge 10 is pushed against subject M. Thereby, elastic body 20 is pushed and crushed, and puncturing tool 12 protrudes from the tip of elastic body 20 to puncture subject M.

As shown in Fig. 5C, when the force which pushes biosensor cartridge 10 is weakened, elastic body 20 returns to its original state (state of Fig. 5A) by a restoring force. Thus, puncturing tool 12 slips out of subject M. Blood D which has come out of subject M is transmitted between raised portion 26 of through hole 22, and puncturing tool 12, so that blood D can be collected.
At this times, within through hole 22 including a puncture hole, the grooves 28 are influenced by deformation of elastic body 20, but they are not necessarily crushed and closed completely. The grooves just have to become at least negative pressure by deformation. Thereafter, through hole 22 is opened to the atmosphere as ventilation passages by grooves 28 are formed by restoration of elastic body 20.

Additionally, since the inner peripheral surface of through hole 22 is subjected to hydrophilic treatment, blood D is collected from sample collection opening 13 along the inner peripheral surface of through hole 22 by the surface tension and capillary phenomenon thereof. Collected blood D is introduced into hollow reaction portion 15. At this time, since sample collection opening 13 is located within enclosed half-open space 23 along with a puncture hole formed by puncturing tool 12, blood D can be easily and reliably collected without moving biosensor cartridge 10. For this reason, since the biosensor device can be used even in subject M whose eyesight is weakened, and measurement can be performed with a small amount of blood, a burden on subject when blood is collected can be reduced. Additionally, since enclosed half-open space 23 is shut off from external air, coagulation of blood D is delayed, thereby facilitating collecting.

If a predetermined amount of blood has been collected, biosensor device 30 is separated from subject M, and waits for a test result to be displayed on display unit 35. Blood D introduced into hollow reaction portion 15 reacts with reagent 14, and data of a current value or charge value (charge amount) measured by detecting electrodes 18a and 18b is sent to control device 33. A calibration curve data table is stored in control device 33, and calculation of blood sugar level is executed on the basis of the measured current value (charge value). If the calculation is ended, a test result is displayed on display unit 35, for example, the blood sugar level can be expressed as a numerical value. Finally, although biosensor cartridge 10 is detached from measuring instrument 31, elastic body 20 returns to almost its original height at this time. Thus, puncturing tool 12 is in a state where it does not protrude from tip 11a of biosensor chip 11. Thereby, a user is not injured by puncturing tool 12, but the used biosensor cartridge 10 can be properly disposed.

Puncturing may be performed using a drive mechanism other than pushing biosensor cartridge 10 against subject M to perform puncturing. The drive mechanism which is used to puncture a subject with the puncturing tool includes a spring, a motor, etc. By using these drive mechanisms, the time taken for puncturing can be shortened and pain at the time of puncturing can be alleviated.

In addition, if the blood collecting burden of a subject is taken into consideration, it is preferable that the volume of hollow reaction portion 15 be equal to or less than 1 µL (microliter), especially, equal to or less than 300 nL (nanoliter). If hollow reaction portion 15 is minute in this way, a sufficient amount of blood of the subject can be collected even if the diameter of puncturing tool 12 is small. Preferably, the diameter is equal to or less than 1000 µm.

As described above, according to the aforementioned biosensor cartridge 10 and biosensor device 30, when tip portion 11a of biosensor chip 11 is pushed against subject M, elastic body 20 is compressed, and puncturing tool 12 for puncturing protrudes. Thus, subject M can be punctured. Additionally, if a pushing force is weakened, puncturing tool 12 is extracted from subject M by the restoring force of elastic body 20, and the blood D flows out of the puncture hole. In this case, since the puncture hole, and sample collection opening 13 provided at tip 11a of biosensor chip 11 are contained in enclosed half-open space 23 of elastic body 20, elastic body 20 returns to its original shape after puncturing. Additionally, since even a small amount of blood D can be easily collected and analyzed by sample collection opening 13, a burden on subject M can be reduced.

Additionally, puncturing tool 12 is prevented from protruding from tip face 21 of elastic body 20 before use, so that protection of puncturing tool 12 and protection of a user can be achieved. Additionally, even when being discarded after use, puncturing tool 12 is prevented from protruding from tip face 21 of elastic body 20, so that the tool can be safely and properly disposed.

Additionally, puncturing and sample collecting can be performed by a series of operation, collecting of a sample can be easily and reliably ensured without being required to align sample collection opening 13 of the biosensor chip with the puncture hole unlike a related art. Additionally, information on blood D is transmitted to measuring instrument 31 via detecting electrodes 18a and 18b, so that measurement can be easily performed in a short time, and a burden on subject M can be reduced.

In addition, the biosensor cartridge of the invention is not limited to the aforementioned embodiment, and proper modifications, improvements, etc. can be made.
For example, although the case where puncturing tool 12 is provided inside biosensor chip 11, i.e., is provided in spacer layer 17 sandwiched between both substrates 16a and 16b has been illustrated in the aforementioned embodiment, biosensor cartridge 10 of the invention is not limited thereto.

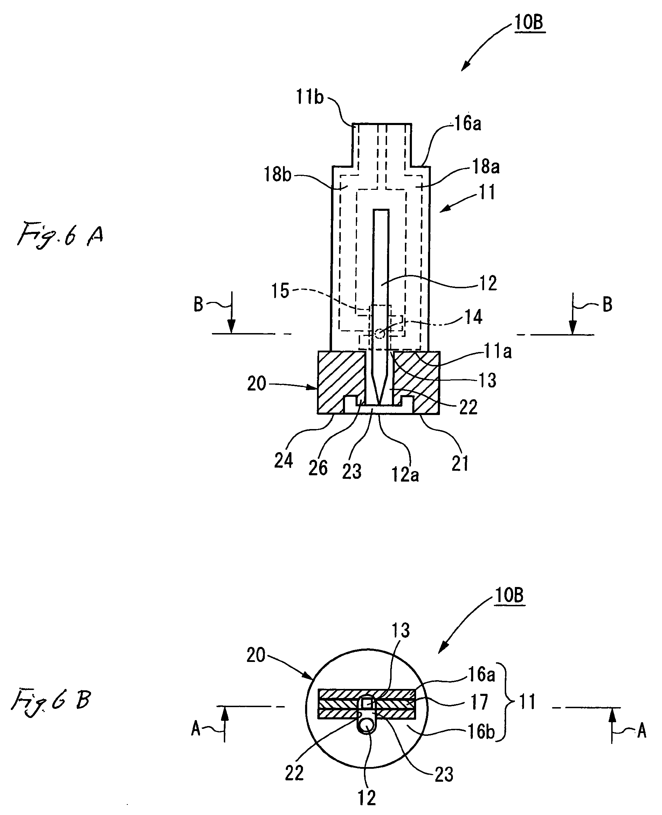

For example, as shown in Figs. 6A and 6B, puncturing tool 12 can also be provided along the outside surface of one substrate 16a. In the case of biosensor cartridge 10B, thin biosensor cartridge 10 can be formed by reducing the thickness of biosensor chip 11. In this regard, since puncturing tool 12 and sample collection opening 13 are somewhat separated from each other, it is desirable to make the cross-sectional shape of through hole 22 oval or the like, thereby making as small as possible a gap formed between the outer peripheral surface of puncturing tool 12 and the inner peripheral surface of through hole 22 of elastic body 20. In addition, in Fig. 6, parts common to those of biosensor cartridge 10 which have been already described are denoted by the same reference numerals, and overlapping description is omitted.

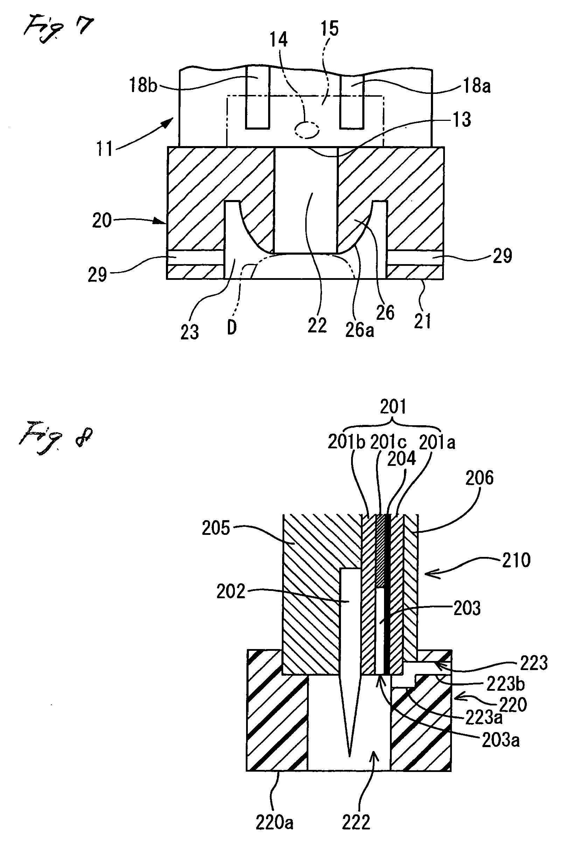

Additionally, although the case where elastic body 20 are provided with two grooves 28 has been described in the aforementioned embodiment, the number of grooves is not limited thereto, and a configuration as shown in Fig. 7 can also be adopted.

The configuration shown in Fig. 7 is a configuration in which a side surface of elastic body 20 is provided with open through hole 29 that is a ventilation passage. Additionally, the enclosed half-open space 23 has a configuration in which raised portion 26 which is formed as a wall surface around through hole 22 is raised similarly to the aforementioned embodiment. However, in this embodiment, the enclosed half-open space has a configuration, in which outer peripheral wall surface 26a of raised portion 26 is suitably inclined (is inclined and curved in this embodiment). That is, the enclosed half-open space has a configuration in which the tip face of raised portion 26 is sharpened. According to such configuration, blood D can be more effectively guided to through hole 22.

Moreover, the case where blood D is collected by the surface tension and capillary phenomenon thereof has been described in the aforementioned embodiment. However, as for adaptation of the invention, a device such as a pump which sucks up blood D which has flowed into puncture hole can be used. Additionally, the detecting electrodes 18a and 18b may be not L-shaped, but straight as shown in Fig. 7.

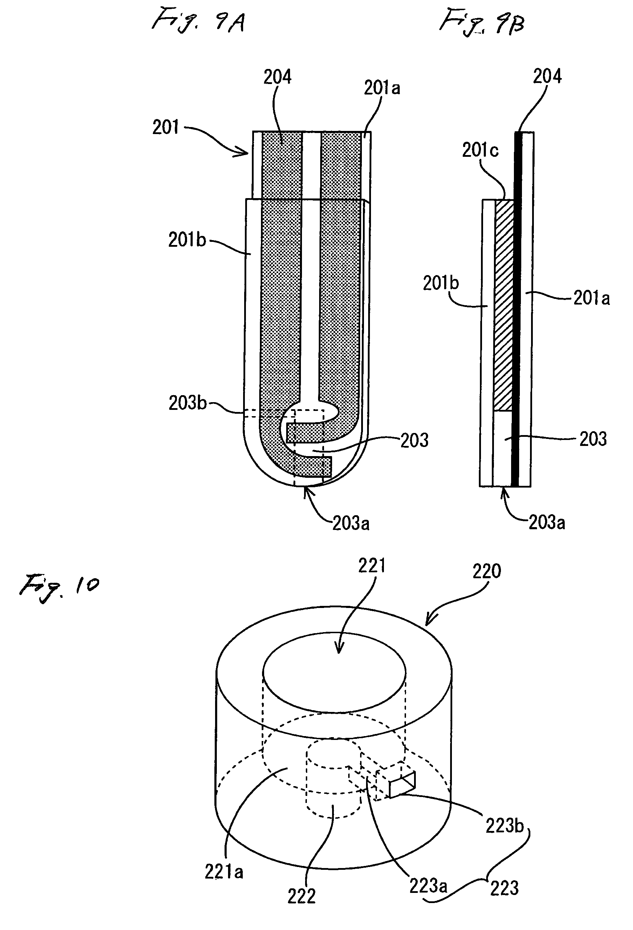

Next, one embodiment of a needle integral sensor of the invention will described with reference to the drawings.
Fig. 8 is a cross-sectional view of the needle integral sensor of this embodiment.
In the needle integral sensor of this embodiment, flow passage forming body 220 as shown in Fig. 10 is attached to needle integral sensor body 210 to which puncturing needle 202 is anchored so that the tip of the puncturing needle protrudes onto one side of flat plate-like detecting portion 201 as shown in Fig. 9, and puncturing needle 202 is inserted into through hole 222 in the middle of flow passage forming body 220.

Flat plate-like detecting portion 201 is formed by sticking two electrically insulating substrates 201a and 201b together with an adhesive or the like. In Figs. 8 and 9, 201c represents an adhesive portion. A square portion to which the adhesive is not applied exists at the tip of detecting portion 201, and the square adhesive non-application portion forms reaction portion 203 which houses a liquid sample such as blood. Additionally, the fine striped adhesive non-application portion exists from reaction portion 203 to a side end of insulating substrate 201a, and this adhesive non-application portion forms air hole 203b which allows reaction portion 203 and the outside of flat plate-like detecting portion 201 to communicate with each other. In Fig. 9, 203a represents an inlet of reaction portion 203.

A pair of detecting electrode patterns 204 is printed on the surface of insulating substrate 201a to which an adhesive is applied, and a pair of electrode patterns 204 are drawn so as to intersect reaction portion 203. Additionally, a reagent which reacts with a liquid sample such as blood is applied to reaction portion 203. Accordingly, the liquid sample contained in reaction portion 203 react with the reagent, so that a change in electric potential or current caused by a chemical change can be detected by the pair of electrodes 204. For example, when blood as the liquid sample is contained in reaction portion 203, an electrical change caused by reaction with the reagent is detected by electrodes 204, so that desired characteristics such as blood sugar level can be detected by a measurement unit.

Puncturing needle 202 is anchored by stacking a needle supporting substrate 205 on the surface (often referred to as the "outside surface of an insulating substrate") of substrate 201b to which an adhesive is not applied. As the puncturing needle, a hollow needle used for a general syringe, a solid needle whose tip is sharp, a lancet needle, etc. can be used. Additionally, mounting portion 206 for mounting needle integral sensor body 210 on a measuring device is stacked on the outside surface of insulating substrate 201a, to which puncturing needle 202 is not attached, of two substrates 201a and 201b of flat plate-like detecting portion 201.

Flow passage forming body 220 is made of an elastic body or a viscoelastic body which can be deformed by a pressing force in a puncturing direction, and be restored to its substantially original shape by release of pressure. Specifically, crude rubber; synthetic rubber such as synthetic isoprene rubber, styrene rubber, nitrile rubber, chloroprene rubber, and acrylic rubber; rubber-like elastic body such as silicone rubber and urethane rubber; thermoplastic elastomer such as ethylene-vinylacetate copolymer; and sponge such as polystyrene foam can be used.

Fitting portion 221 for fitting tip portion of sensor body 210 is recessed at the side of flow passage forming body 220 attached to the needle integral sensor body 210. Through hole 222 penetrating from bottom face 221a of fitting portion 221 to subject abutting surface 220a of flow passage forming body 220 is provided substantially in the center of fitting portion 221 that is a sensor body attachment portion, and the tip of puncturing needle 202 is inserted into through hole 222. In fitting portion bottom surface 221a, groove 223a is recessed in a radial direction from a position which becomes the vicinity of reaction portion inlet 203a, and communication hole 223b which passes through the peripheral wall surface of flow passage forming body 220 is bored in a position slightly above the groove 223a. Ventilation passage 223 which allows through hole 222 and the outside of flow passage forming body 220 to communicate with each other is formed by combination of groove 223a and communication hole 223b, and the position where the groove 223a and the communication hole 223b are connected together is formed as a curved passage which becomes a curved portion.

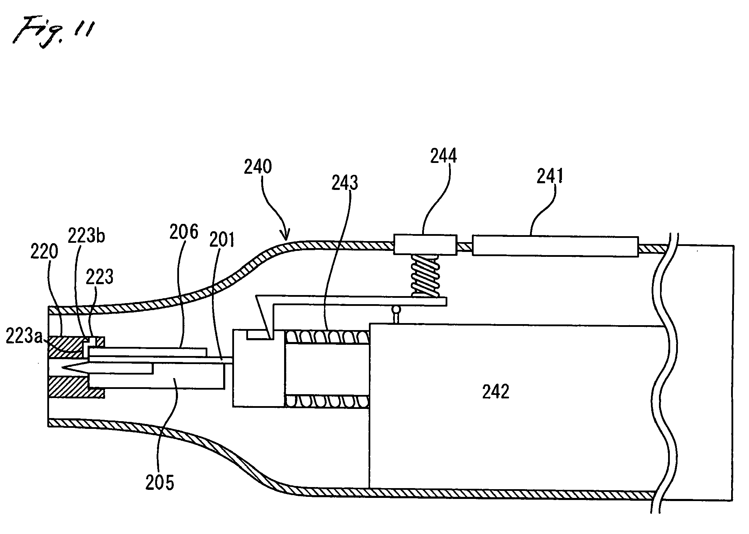

The needle integral sensor having the configuration as described above is mounted on measuring instrument 240 including display unit 241, measurement unit 242, spring 243 for puncturing, and spring-operated button 244, as shown in Fig. 11. At the time of mounting, spring 243 for puncturing is set in a compressed state by inserting mounting portion 206 of needle integral sensor body 210 into a set portion (not shown) of the measuring instrument. By pushing spring-operated button 244, spring 243 is released from the compressed state, so that needle integral sensor body 210 can be driven in the puncturing direction.

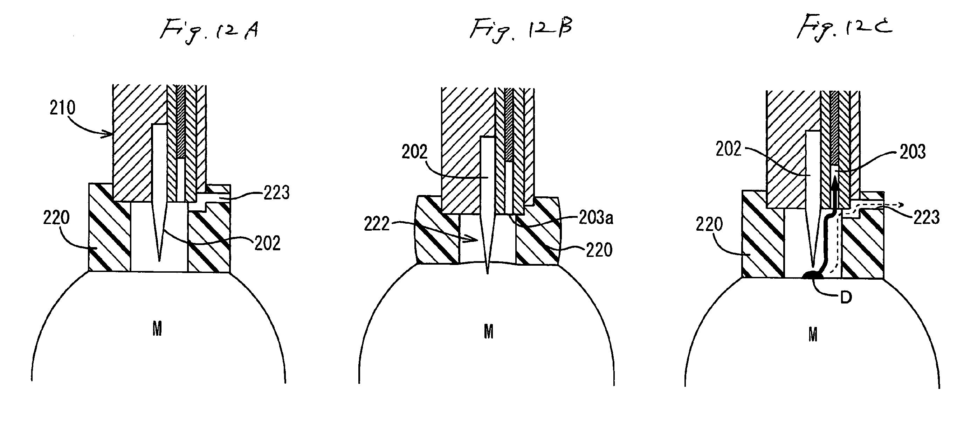

When the bottom surface of flow passage forming body 220 is pushed against skin M of a subject, for example, with a finger in the compressed state (spring-biased state) of the spring for puncturing (refer to Fig. 12A), thereby operating a button in a direction in which the spring holding puncturing needle 202 extends, needle integral sensor body 210 is pushed out toward skin M. Along with this, a pressing force is generated in the puncturing direction in flow passage forming body 220 attached to the tip of needle integral sensor body 210, whereby flow passage forming body 220 is deformed so as to be compressed in the puncturing direction or expanded in a radial direction. Since puncturing needle 202 attached to the tip of needle integral sensor body 210 is pushed out toward skin M in a deformed state of flow passage forming body 220, puncturing needle 202 protrudes from subject abutting surface 220a of flow passage forming body 220, and skin M is punctured (refer to Fig. 12B).

Next, when the biasing by the spring is released, flow passage forming body 220 returns to its substantial original shape by its own restoring force, and along with this, puncturing needle 202 is pulled out of skin M and is housed in flow passage 222 (refer to Fig. 12C). Blood D as a liquid sample discharged to the surface of skin D by the puncturing rises along the needle or along the internal wall surface of through passage 222 (shown by a thick line arrow in Fig. 12C).

On the other hand, by opening or restoration of ventilation passage 223 by restoration of flow passage forming body 220, the atmosphere not only can flow in from the outside of flow passage forming body 220, but the air within through hole 222 can be exhausted with the rise in blood D (shown by a broken line arrow in Fig. 12C). The blood which has risen along puncturing needle 202, or the blood which has risen along the wall surface of through hole 222 rises even to fitting portion bottom surface 221a, and then flows toward groove 223a along the flow of air. The flow of blood D goes via reaction portion inlet 203a, and is contained within reaction portion 203 from inlet 203a.
Additionally, since ventilation passage 223 becomes a curved passage, flow of a liquid with a higher viscosity compared with air is prevented in a curved portion. Accordingly, the blood which has risen along the wall surface of through hole 222 near groove 223a can also be kept from flowing out from ventilation passage 223, and can be expected to flow toward reaction portion inlet 203a.

As described above, in the needle integral sensor of this embodiment, discharged blood can be effectively introduced into reaction portion 203 irrespective of whether puncturing needle 202 and reaction portion inlet 203a are in positions separated from each other. Thus, only a minimum amount of blood required for measurement may be discharged. Accordingly, the portion which pierces a skin by the puncturing needle is shallow, and it is possible to alleviate a pain on a subject more than a conventional lancet type measuring device in which a puncturing needle is driven separately from a sensor body.

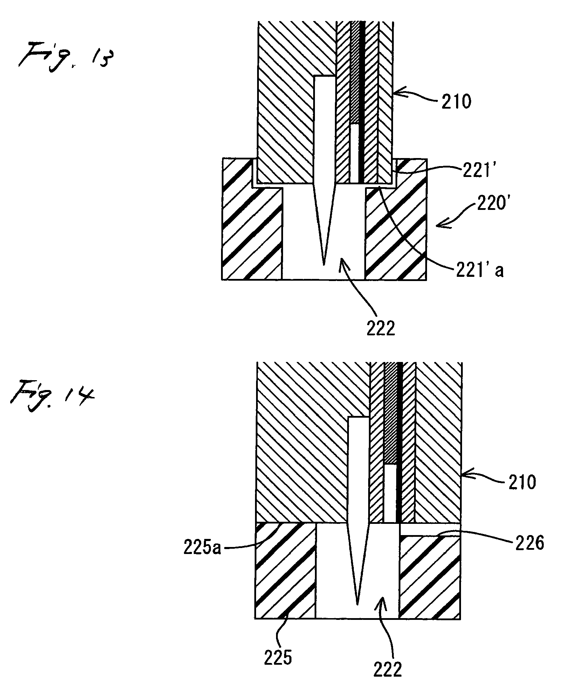

In addition, in the above embodiment, ventilation passage 223 is composed of groove 223a provided in fitting portion bottom surface 221a, and communication hole 223b bored in an peripheral wall portion which becomes an outer fitting portion of the sensor body in flow passage forming body 220. However, the ventilation passage formed in the sensor body attachment portion is not limited thereto. For example, instead of groove 223a, a portion of fitting portion bottom surface 221a may cut out to allow through hole 222 and communication hole 223b to communicate with each other, and fitting portion bottom surface 221a may only be roughened to such a degree that air can pass therethrough. Additionally, the ventilation passage provided in fitting portion bottom surface 221a, and the outside of the flow passage forming body may be allowed to communicate with each other by increasing the clearance of the fitting portion instead of the communication hole 223b. For example, in flow passage forming body 220' used for the needle integral sensor shown in Fig. 13, a fine groove is provided in fitting portion bottom surface 221'a in fitting portion 221' which becomes a sensor body attachment portion to such a degree that air can pass therethrough, and fitting of sensor body 210 is designed to a loosely fitting state, thereby forming a ventilation passage which allow through hole 222 and the outside of flow passage forming body 220' to communicate with each other.

Additionally, in the embodiment shown in Fig. 8, even if a tip portion of mounting portion 206 is cut out in the portion corresponding to the curved portion of ventilation passage 223, and thereby, sensor body 210 and flow passage forming body 220 are attached by fitting with no substantial clearance therebetween, the communication between groove 223a and communication hole 223b is secured. However, cut-out of mounting portion 206 is not indispensable in formation of the curved ventilation passage. On the other hand, by cutting out mounting portion 206 to a position above the fitting portion 221, a ventilation passage which communicates with the outside of flow passage forming body 220 may be formed by groove 223a formed in fitting portion bottom surface 221a, and the cutout portion of the mounting portion 206.

Additionally, in the embodiment shown in Fig. 8, air hole 203b is provided above the reaction portion 203. However, in the needle integral sensor of the invention, air hole 203b may not be provided as long as a configuration in which a liquid sample which has risen along through hole 222 is preferentially introduced into reaction portion inlet 203a is provided. The configuration in which a liquid sample which has risen along through hole 222 is preferentially introduced into reaction portion inlet 203a may be a configuration in which the ventilation passage is provided with a curved portion, and a liquid flows with difficulty even if air flows, or a surfactant may be only applied in the vicinity of the inlet of the reaction portion.

Additionally, the fitting type flow passage forming body which is fitted and attached to the sensor body has been described has been described in the above embodiment. However, the flow passage forming body used in the invention is not limited thereto. For example, as shown in Fig. 14, cylindrical flow passage forming body 225 having through hole 222 serving as a flow passage provided so as to pass through the middle thereof may be anchored and attached to sensor body 210 with an adhesive or the like. In the flow passage forming body 225, the ventilation passage is groove 226 provided in sensor body attachment surface 225a so as to extend in the radial direction.

Moreover, in the above embodiment, the reaction portion inlet directly communicates with the through hole. However, the reaction portion inlet may communicate with the through hole serving as a flow passage by arranging the reaction portion inlet midway of the ventilation passage. In this case, a liquid outflow preventing mechanism of the curved portion or the like may be provided in a ventilation passage portion from the reaction portion inlet to the outside of the flow passage forming body such that a liquid sample which flows through the ventilation portion flows into the reaction portion from the reaction portion inlet more preferentially than flowing through the inside of the ventilation passage and flowing out to the outside of the flow passage forming body, or a surfactant may be applied to the vicinity of the reaction portion inlet such that the liquid sample easily flows into the reaction portion more preferentially than flowing through the ventilation passage.

In the needle integral sensor of the invention, the position in which the ventilation passage is provided is not limited to the sensor body attachment portion. The ventilation passage has only to be provided so as to allow the through hole serving as a flow passage and the outside of the flow passage forming body to communicate with each other. For example, the ventilation passage may be a communication hole formed by perforating the peripheral wall of the flow passage forming body in a position midway of the through hole.

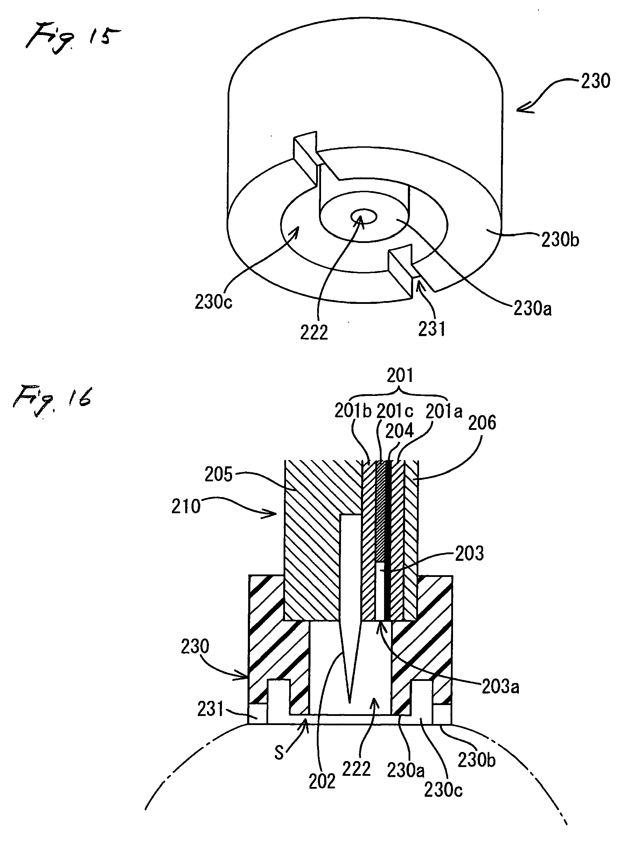

Additionally, the ventilation passage may be formed in the subject abutting surface of the flow passage forming body, and has only to be provided so as to allows the through hole and the outside of the flow passage forming body to communicate with each other in a state of abutting on a subject. In a case where the ventilation passage is provided in the abutting surface of subject, it is preferable to provide a liquid outflow preventing mechanism which prevents a liquid sample discharged to the surface of a subject from leaking out to the outside of the flow passage forming body from the ventilation passage. In the above embodiment, a liquid is prevented from flowing out to the outside of the flow passage forming body by providing the curved portion midway of the ventilation passage. However, a protective wall which prevents outflow of a liquid may be provided separately from the peripheral wall which forms the through hole. The flow passage forming body having such a configuration includes, for example, flow passage forming body 230 which has a double wall structure as shown in Figs. 15 and 16.

Flow passage forming body 230 has a double wall structure of internal wall 230a which constitutes through hole 222 serving as a flow passage, and external wall 230b that is a protective wall against liquid outflow, and serves as the peripheral wall of flow passage forming body 230. The surface of internal wall 230a which abuts on a subject is made slightly lower than the surface of external wall 230b which abuts on a subject. Thereby, in a state where flow passage forming body 230 is simply made to abut on the subject (shown by a one-dot chain line shown in Fig. 16), the surface of external wall 230b which abuts on the subject becomes a subject abutting surface, and gap S is formed between internal wall 230a and the subject. In a state where cutout portion 231 is cut out in the subject abutting surface of external wall 230b, and flow passage forming body 230 abuts on the subject, through hole 222 and the outside of flow passage forming body 230 communicate with each other via cutout portion 231, and gap S formed between internal wall 230a and the subject abutting surface. That is, the ventilation passage is constituted by cutout portion 231, valley portion 230c between internal wall 230a and external wall 230b, and gap S formed between internal wall 230a and the subject abutting surface. In the needle integral sensor including such a flow passage forming body 230, flow passage forming body 230 is compressed by application of pressure in the puncturing direction, and the tip of the puncturing needle protrudes from through hole 222, and pieces a skin. In this state, the surface of internal wall 230a on the side of the subject abuts on the subject, and through hole 222 is brought into a substantially sealed state. By release of a pressing force, flow passage forming body 230 is restored, gap S is formed between internal wall 230a and the subject, and through hole 222 and the ventilation passage are brought into a communication state. In this state, inflow and outflow of air can occur between outside of flow passage forming body 230 and through holes 222 via the ventilation passage. Inflow of air from the subject abutting surface returns the inside of the flow passage forming body 222 to atmospheric pressure, and thereby, discharge of blood when the puncturing needle is pulled out from the subject, can be expected to be promoted. The blood discharged to the surface of a skin can be guided to reaction portion inlet 203a through the inner peripheral surface of internal wall 230a that is the peripheral wall of puncturing needle 202 and through hole 222. Even in this case, rise of blood through the inside of through hole 222 can be expected to be promoted by inflow and outflow of air by the ventilation passage, especially, inflow of air from the subject abutting surface. On the other hand, the external wall 230b prevents the blood discharged to the surface of a skin from flowing out of flow passage forming body 230. In addition, in a case where the ventilation passage is provided on the side of the subject abutting surface, not only the liquid outflow preventing mechanism may be provided, but a surfactant may be applied to the vicinity of the opening of internal wall 230a on the side of the subject, serving as an inlet of through hole 222, so that a liquid sample flow into through hole 222 more preferentially than valley portion 230c.

In addition, in the needle integral sensor of the invention, the ventilation passage provided in the flow passage forming body is not limited to one, and a plurality of ventilation passages may be provided as long as a liquid sample discharged by puncturing can be effectively contained in the reaction portion. For example, in flow passage forming body 230, the ventilation passage may be provided even in the sensor body attachment portion as shown in the embodiment of Fig. 8, separately from the ventilation passage provided on the side of the subject abutting surface.

Additionally, in the above embodiment, the flow passage forming body is composed of an elastic body or a viscoelastic body, and puncturing and pull-out of the needle is performed using deformation or restoration of the flow passage forming body. However, the needle integral sensor of the invention is not limited thereto. The flow passage forming body may be composed of a rigid body. In this case, the measuring instrument on which the needle integral sensor is mounted may be provided with a drive mechanism which pulls back the needle along with the drive mechanism which drives the needle integral sensor body in the puncturing direction so that the puncturing needle can project or retreat from the flow passage forming body.

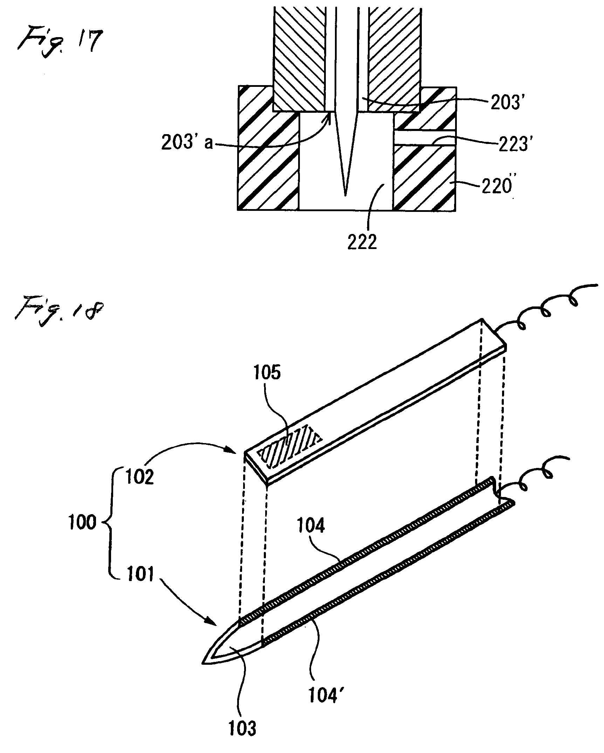

Additionally, in the above embodiment, the puncturing needle is attached to the outside of the reaction portion. However, in the needle integral sensor of the invention, as shown in Fig. 17, the puncturing needle may be attached and fixed to the inside of reaction portion 203'. In addition, in flow passage forming body 220" used for the needle integral sensor shown in Fig. 17, ventilation passage 223' is provided midway of through hole 222.

Moreover, in the embodiment shown in Fig. 8, the electrodes are provided on the insulating substrate on the side where the needle is not attached. However, in the needle integral sensor of the invention, the positional relationship between a needle attachment position and an electrode substrate is not limited. The electrodes may be provided on a substrate on the side where the needle is attached, and a positive electrode and a negative electrode do not need to be provided in the same substrate. A positive electrode may be provided on one substrate, and a negative electrode may be provided on the other substrate. Additionally, although the detecting portion is configured such that two substrates are stuck together, for example, as disclosed in Pamphlet of International Publication No. 05/010519, the detecting portion may be configured such that a pair of electrodes is arranged on one substrate, and the electrodes are bent inward. Additionally, the shape of the detecting portion may be not only a flat plate shape but a cylindrical type, and the reaction portion may also be cylindrical.

In addition, although the biosensor cartridge and needle integral sensor according to the invention are expressed differently, they are common in that the puncturing needle and the biosensor chip are integrated.
Additionally, both the flow passage forming body and the elastic body are common to each other in that they are disposed at the puncturing needle and the tip of the biosensor chip.
Additionally, the sensor body and the biosensor chip can be understood as a synonym.

### Industrial Applicability

As described above, in the biosensor cartridge according to the invention, the tip of the biosensor chip is provided with the elastic body. Thus, the puncturing tool is pulled out by the restoring force of the elastic body after puncturing. Also, an airflow can be used for sample collection when a sample collection space formed by the elastic body changes to release to the atmosphere from a negative pressure state. Thus, even a small amount of sample can be easily and very surely collected by sample collection opening, and the biosensor cartridge is useful as a biosensor cartridge which conducts measurement and analysis of a chemical substance using a reagent contained in a hollow reaction portion of a chip. Additionally, according to the invention, a method of using a biosensor cartridge and a biosensor device capable of carrying out a reliable test can be provided.

Although the invention has been described in detail with reference to the specific embodiments, it is clear to those skilled in the art that various alternations and modifications can be made without departing from the spirit and scope of the invention. The present application is based on Japanese Patent Application (JP2006-341792) filed on December 19, 2006, and Japanese Patent Application (JP2007-035931) filed on February 16, 2007, the entire contents of which are incorporated herein by reference.

## Claims

1. A biosensor cartridge comprising:
a biosensor chip, and
a puncturing tool fixed to a portion of the biosensor chip and having a tip protruding therefrom, wherein
an elastic body having a through hole which contains a sample collection opening provided at a tip of the biosensor chip and a puncture hole formed in a subject by the puncturing tool is provided at a tip of the biosensor chip, and
the elastic body includes a ventilation passage which passes through between an internal wall surface which forms the through hole and an external wall surface of the elastic body.

2. The biosensor cartridge of Claim 1, wherein
the sample collection opening provided at the tip of the biosensor chip and the puncture hole formed in the subject by the puncturing tool are connected together by the through hole.

3. The biosensor cartridge of Claim 1 or 2, wherein
the elastic body is configured such that the internal diameter of the hole on the side which touches the subject is made larger than the internal diameter of the hole on the side which touches the biosensor chip.

4. The biosensor cartridge of Claim 3, wherein
the elastic body is configured such that the internal wall surface which forms the through hole is raised toward the tip of the elastic body on the side which touches the subject.

5. The biosensor cartridge of any one of Claims 1 to 4, wherein
the ventilation passage is a groove provided at the end face of the elastic body on the side of the subject.

6. The biosensor cartridge of any one of Claims 1 to 4, wherein
the ventilation passage is an open through hole formed substantially along a radial direction of the elastic body.

7. The biosensor cartridge of any one of Claims 1 to 6, wherein
a plurality of the ventilation passages are formed in the elastic body, and are arranged so as to be symmetrical in the radial direction of the elastic body with the axial center of the elastic body as a center.

8. The biosensor cartridge of any one of Claims 1 to 7, further comprising:
a drive mechanism which punctures a subject with the puncturing tool.

9. The biosensor cartridge of any one of Claims 1 to 8, wherein
the elastic body has stickiness.

10. The biosensor cartridge of any one of Claims 1 to 9, wherein
the elastic body is fixed to the tip of the biosensor chip with its own stickiness, fixed to the tip of the biosensor chip with an adhesive, or fixed to the tip of the biosensor chip with a double-sided tape.

11. A method of using a biosensor cartridge of any one of Claims 1 to 10, the method comprising the steps of:
pushing the elastic body against the subject to compress the elastic body to puncture the subject,
pulling out a puncturing tool from the subject by a restoring force of the elastic body, and
performing sample collection while the through hole is opened to the atmosphere.

12. A biosensor device comprising:
the biosensor cartridge of any one of Claims 1 to 10, and
a measuring instrument which is connected to detecting electrodes of the biosensor cartridge and obtains information on a sample which has been collected.

13. A needle integral sensor comprising:
a needle integral sensor body integrally having a puncturing needle which discharges a liquid sample from a subject, a reaction portion which contains the liquid sample, and a detecting portion which detects the result of the reaction portion; and
a flow passage forming body in which a through hole serving as a flow passage from an abutting portion of the subject to the reaction portion is provided, and in which the puncturing needle is inserted into the through hole, wherein
the flow passage forming body is formed with a ventilation passage which communicates the through hole and the outside of the flow passage forming body with each other.

14. The needle integral sensor of Claim 13, wherein
the flow passage forming body is an elastic body or viscoelastic body which enables the puncturing needle to project and retract from the through hole by deformation.

15. The needle integral sensor of Claim 13 or 14, wherein
the ventilation passage is provided in an attachment portion of the needle integral sensor body.

16. The needle integral sensor of Claim 15, wherein
the ventilation passage is provided so that the reaction portion inlet is located midway of the ventilation passage or in the vicinity of the ventilation passage inlet.

17. The needle integral sensor of any one of Claims 13 to 16, wherein
the ventilation passage is provided with a liquid outflow preventing mechanism.

18. The needle integral sensor of Claim 17, wherein
the liquid outflow preventing mechanism is a curved portion provided at a midway of the ventilation passage.

19. The needle integral sensor of any one of Claims 13 to 18, wherein
a surfactant is applied at least in the vicinity of an inlet of the reaction portion.
